# FASCICULE DE BREVET EUROPEEN

(11) **EP 1 704 851 B1**
(45) Date de publication et mention de la délivrance du brevet: **13.02.2008**
(21) Numéro de dépôt: 06111015.1
(22) Date de dépôt: 13.03.2006
(51) Int. Cl.: A61K 8/365, A61K 8/06, A61K 8/04, A61Q 19/10, A61Q 1/14, A61Q 5/02, C11D 1/06

(54) **Emulsion H/E moussante et son utilisation dans le domaine cosmétique**
Schäumende O/W-Emulsion und ihre Verwendung in der Kosmetik
Foaming O/W emulsion and its cosmetic use

(30) Priorité: 25.03.2005 FR 0550781
(43) Date de publication de la demande: 27.09.2006
(73) Titulaire: L'OREAL, S.A., 75008 Paris (FR)
(72) Inventeur: Sebillotte-Arnaud, Laurence, 94240, L'hay les Roses (FR); Aubrun, Odile, 92160 Antony (FR); De Salvert, Armelle, 75013, Paris (FR)
(74) Mandataire: Rasson, Catherine

(56) Documents cités:
- EP-A- 0 291 207
- EP-A- 0 793 955
- EP-A- 1 029 535
- DE-A1- 4 318 171
- FR-A- 2 733 417
- US-B1- 6 368 584

## Description

La présente invention se rapporte à une composition moussante pour application topique, notamment cosmétique et/ou dermatologique, sous forme d'émulsion huile-dans-eau, riche en huile, susceptible d'être obtenue par inversion de phase, et à ses utilisations notamment dans le domaine cosmétique ou dermatologique.

Le nettoyage de la peau est très important pour le soin du visage, et il doit être le plus performant possible car les résidus gras tels que l'excès de sébum, les restes des produits cosmétiques utilisés quotidiennement et les produits de maquillage notamment les produits « waterproof » résistants à l'eau, s'accumulent dans les replis cutanés et peuvent obstruer les pores de la peau et entraîner l'apparition de boutons.

Dans le domaine du nettoyage, il est connu d'utiliser des gels aqueux moussants. Leur action nettoyante est apportée par les tensioactifs qu'ils contiennent, ces tensioactifs mettant en suspension les résidus gras et les pigments des produits de maquillage. Toutefois, ces gels ont souvent l'inconvénient de laisser la peau sèche et de donner des sensations de tiraillements. De plus, leur pouvoir démaquillant n'est pas toujours suffisant.

Par ailleurs, dans le domaine du démaquillage, il est connu d'utiliser des laits ou des crèmes (produits non moussants). Ces produits sont plus confortables que les gels aqueux mais ils ont souvent l'inconvénient de laisser un résidu trop gras après application sur la peau, et il est alors nécessaire d'utiliser un tonique pour enlever le surplus de corps gras résiduel.

De ce fait, on cherche à faire des produits deux en un, qui soient à la fois moussants et nettoyants tout en apportant une valence soin et/ou qui démaquillent sans laisser de résidu trop gras.

Un des moyens pour apporter cette valence soin dans les produits moussants est d'y incorporer de l'huile et de préparer donc des émulsions. Des produits de ce type existent déjà, et ils peuvent être utilisés en tant que nettoyants de soin aussi bien pour le visage que pour le corps puisqu'ils laissent un peu d'huile à la surface de la peau, ou bien comme produit démaquillant à rincer. Toutefois, ces produits ne donnent pas satisfaction en ce qui concerne le démarrage de la mousse et/ou le volume de mousse obtenu car la présence d'huiles entrave le bon développement de la mousse. En outre, la quantité d'huiles y est en général limitée du fait justement des problèmes posés par la présence d'huile pour le développement de la mousse.

Ainsi, le document FR-A-2,733,417 décrit des émulsions huile-dans-eau moussantes sous forme de crèmes, comprenant des tensioactifs non-ioniques moussants, des huiles ayant des paramètres de solubilité déterminés, et des polymères réticulés notamment vinyliques comme agents gélifiants. Toutefois, ces compositions présentent l'inconvénient de ne pas donner une mousse satisfaisante, et notamment d'avoir un mauvais démarrage de mousse et de donner un faible volume de mousse.

Le document EP-A-0 793 955 décrit une émulsion H/E ultrafine moussante comprenant une huile cosmétique, un émulsionnant non-ionique de HLB 9-18 et un tensioactif moussant, préférentiellement préparée par la méthode d'inversion de phase. La composition peut contenir 0.5-50% d'huile. En tant que tensioactifs moussants, on peut utiliser des anioniques moussants tels que les alkyléther carboxylates.

II subsiste donc le besoin de réaliser des produits qui, tout en étant aussi doux que les produits non moussants que sont les crèmes ou les laits, aient de bonnes propriétés moussantes avec une bonne qualité de mousse, tout en ayant une bonne tolérance et en contenant une grande quantité d'huile.

II a été trouvé de manière surprenante qu'il était possible de répondre au problème posé en utilisant une émulsion ayant des petites tailles de gouttelettes de phase huileuse, contenant l'association d'au moins un alkyl polyglucoside et d'au moins un acide alkyl glycol carboxylique, en tant que tensioactifs moussants, cette émulsion étant riche en huiles et pouvant être notamment obtenue par inversion de phase en température. Les compositions obtenues possèdent de meilleures qualités de mousse par rapport à l'état de la technique et de bonnes propriétés de tolérance.

Ainsi, les compositions obtenues sont très douces et peuvent être appréciées par tout type de peaux et en particulier par les peaux sèches et sensibles.

La présente invention a donc pour objet une composition moussante pour application topique sous forme d'émulsion huile-dans-eau, comprenant une phase huileuse dispersée dans une phase aqueuse, caractérisée en ce que :
- la taille des gouttelettes de la phase huileuse D[4,3] est inférieure ou égale à 4 µm,
- la phase huileuse (A) est présente en une quantité d'au moins 30 % en poids par rapport au poids total de la composition,
et en ce que la composition contient :
- un système émulsionnant (B) ayant un HLB allant de 8 à 18, présent en une quantité d'au moins 2 % en poids par rapport au poids total de la composition,
- un système moussant (C) comprenant au moins un tensioactif moussant choisi parmi les acides alkyl glycol carboxyliques et leurs sels, de formule (I) suivante :

   R₁-CHOH-CH₂-O-CH₂-COO⁻ X⁺ (I)
dans laquelle R₁ désigne un radical alkyle saturé ou insaturé, linéaire ou ramifié, comportant de 8 à 30 atomes de carbone, et X désigne l'hydrogène ou un cation minéral ou organique,
- le rapport en poids phase huileuse (A) / système moussant (C) allant de 1,5 à 12.

Du fait qu'elle est destinée à une application topique, la composition de l'invention contient un milieu physiologiquement acceptable. Par "milieu physiologiquement acceptable", on entend un milieu convenant à une application topique sur la peau ou les phanères, c'est-à-dire compatible avec la peau, les muqueuses, les lèvres, les cils, les yeux, les cheveux et les ongles. Cette composition peut constituer notamment une composition cosmétique ou dermatologique.

On entend par « phase huileuse » dans la présente demande, la phase contenant les composés lipophiles que sont notamment les huiles (constituants lipophiles liquides à température ambiante), les gommes, les pâtes et les cires. Ce sont par exemple des triglycérides, des hydrocarbures, des esters, des éthers, des silicones, comme décrit plus loin, et tous les additifs lipophiles éventuellement présents, sous réserve que ces composés ne nuisent pas à la formation de la mousse. Les émulsionnants et co-émulsionnants du système émulsionnant ne font pas partie de la phase huileuse (A) telle que définie ci-dessus.

Les émulsions H/E selon l'invention comprennent une phase huileuse (ou phase lipophile) dispersée dans une phase aqueuse et elles sont de préférence obtenues par la technologie d'inversion de phase en température et se caractérisent par :
- leur viscosité : il s'agit principalement de crèmes,
- leur pH qui va de 5 à 10,5, de préférence de 5 à 9,
- la petite taille des gouttelettes de la phase huileuse inférieure ou égale à 4 µm,
- leur stabilité : après deux mois à 45°C, il ne se produit ni déphasage macroscopique ni changement de texture tel que l'apparition de grains,
- Leur pouvoir démaquillant important.

Le principe d'émulsification par inversion de phase en température (en Anglais : Phase Inversion Temperature ou PIT) est, dans son principe, bien connu de l'homme de l'art ; il a été décrit en 1968 par K. Shinoda (J. Chem. Soc. Jpn., 1968, 89, 435). II a été montré que cette technique d'émulsification permet d'obtenir des émulsions fines stables (K. Shinoda et H. Saito, J. Colloïd Interface Sci., 1969,30, 258). Cette technologie a été appliquée en cosmétique dès 1972 par Mitsui et al. («Application of the phase-inversion-temperature method to the emulsification of cosmetics» ; T. Mitsui, Y. Machida and F. Harusawa, American. Cosmet. Perfum., 1972, 87, 33).

Le principe de cette technique est le suivant : on réalise le mélange d'une phase aqueuse et d'une phase huileuse, que l'on porte à une température supérieure à la température PIT, température d'inversion de phase du système, qui est la température à laquelle l'équilibre entre les propriétés hydrophile et lipophile du ou des émulsionnants mis en oeuvre est atteint ; à température élevée, c'est-à-dire supérieure à la température d'inversion de phase (>PIT), l'émulsion est de type eau-dans-huile, et, au cours de son refroidissement, cette émulsion s'inverse à la température d'inversion de phase, pour devenir une émulsion de type huile-dans-eau, et ceci en étant passée auparavant par un état de microémulsion. Ce procédé permet d'obtenir facilement des émulsions de diamètre inférieur à 4 µm.

Les émulsions obtenues selon l'invention, malgré la quantité importante d'huiles qu'elles contiennent, sont stables. On entend par « composition stable » une composition qui reste macroscopiquement homogène après 2 mois à 45°C. Une telle stabilité signifie qu'il ne se produit ni déphasage macroscopique ni changement de texture telle que l'apparition de grains, après ce laps de temps.

Les compositions selon l'invention peuvent se présenter sous forme de crèmes plus ou moins épaisses, opaques à translucides, et elles peuvent s'écouler ou non sous leur propre poids selon leur viscosité. La viscosité mesurée à 25°C avec l'appareil de mesure Rheomat 180 à 200 rpm (tours par minute) est égale ou supérieure à 1 Pa.s. Le Rheomat 180 est équipé d'un mobile différent selon les viscosités, par exemple d'un mobile 3 pour la gamme des viscosités de 0,2 à 4 Pa.s, et d'un mobile 4 pour la gamme des viscosités supérieures à 2 Pa.s. Mesurée dans les conditions indiquées ci-dessus, la viscosité des compositions de de l'invention peut aller par exemple de 1 à 200 Pa.s et de préférence de 5 à 180 Pa.s. Cette viscosité est mesurée généralement 10 minutes après la mise en rotation du mobile.

La taille moyenne des gouttelettes de phase huileuse et leur polydispersité sont par exemple mesurées par diffraction de la lumière à l'aide d'un granulomètre Mastersizer 2000 (commercialisés par Malvern Instruments). Ces mesures sont effectuées sur l'émulsion diluée dans une solution de SDS (Sodium Dodecylsulfate) à 1 % dans l'eau. Un logiciel permet d'accéder au diamètre moyen en volume D[4,3] (µm) (Voir Operators Guide, Malvern Instruments, Decembre 1998, p.61 à 67).

La taille moyenne D[4,3] (µm) des gouttelettes de phase huileuse de la composition de l'invention est inférieure ou égale à 4 µm et elle varie notamment de 0,09 µm à 4 µm, plus particulièrement de 0,1 µm à 2 µm et de préférence de 0,1 µm à 1 µm.

La polydispersité est ici définie par la déviation absolue (absolute deviation) par rapport à la médiane ou « uniformité ». Cette valeur doit être inférieure à 1.

### Système moussant

Dans la composition selon l'invention, le rapport en poids de la quantité de phase huileuse (A) sur la quantité du système moussant (C) va de 1,5 à 12, de préférence de 4 à 10.

On entend par « système moussant » l'ensemble de tous les tensioactifs moussants. Le système moussant (C) comprend au moins un tensioactif anionique choisi parmi les acides alkyl glycol carboxyliques et leurs sels. Ce système peut comprendre seulement un ou plusieurs tensioactifs anioniques choisis parmi les acides alkyl glycol carboxyliques et leurs sels, ou il peut comprendre un mélange d'un ou plusieurs tensioactifs de ce type et d'autres tensioactifs moussants.

Les tensioactifs moussants sont généralement hydrophiles et donc présents dans la phase aqueuse.

### Acides alkyl glycol carboxyliques

Les acides alkyl glycol carboxyliques (ou acides 2-(2-Hydroxyalkyloxy acétique), et leurs sels sont des tensioactifs moussants de formule (I) suivante :

R₁-CHOH-CH₂-O-CH₂-COO⁻ X⁺ (I)

dans laquelle R₁ désigne un radical alkyle saturé ou insaturé, linéaire ou ramifié, comportant de 8 à 30 atomes de carbone, et X désigne l'hydrogène ou un cation minéral ou organique tel que ceux issus d'un métal alcalin (par exemple Na+, K+), NH4+, les ammoniums issus des aminoacides basiques tels que la lysine, l'arginine, la sarcosine, ou bien encore des amino-alcools tels que la monoéthanolamine, la diéthanolamine, la triéthanolamine, la glucamine, la N-méthyl glucamine, l'amino-3 propanediol-1,2.

Les acides hydroxy-2 alkyl carboxyliques préférés selon la présente invention sont des composés de formule (I) dans laquelle R₁ désigne plus particulièrement un radical alkyle saturé ou insaturé, linéaire ou ramifié comportant de 8 à 18 atomes de carbone.

Parmi les tensioactifs de formule (I), on peut citer notamment le lauryl glycol carboxylate de sodium, commercialisé sous les dénominations BEAULIGHT SHAA® ou BEAULIGHT LCA-25N® par la société SANYO, ou sa forme acide correspondante commercialisée sous la dénomination BEAULIGHT SHAA (Acid Form)® par la société SANYO.

La quantité (en matière active) de tensioactifs anioniques de type alkyl glycol carboxylique peut aller par exemple de 0,5 à 20 % en poids, de préférence de 1 à 15 % en poids, mieux de 2 à 10 % en poids par rapport au poids total de la composition.

### Autres tensioactifs moussants

La composition peut contenir d'autres tensioactifs moussants, qui peuvent être choisis parmi les tensioactifs moussants non-ioniques, amphotères ou zwitterioniques, anioniques et cationiques.

La composition peut ne contenir que de tensioactifs anioniques de type alkyl glycol carboxylique ou bien elle peut contenir un mélange de tensioactifs moussants dont les tensioactifs anioniques de type alkyl glycol carboxylique. La quantité totale en tensioactifs moussants (en matière active) peut aller par exemple de 0,5 à 20 % en poids, de préférence de 1 à 15 % en poids et mieux de 5 à 15 % en poids par rapport au poids total de la composition.

Comme tensioactifs moussants non ioniques, la composition peut contenir par exemple, un ou plusieurs tensioactifs non ioniques choisis parmi les alkyl polyglucosides (APG), les esters de maltose, les alcools gras polyglycérolés, les dérivés de glucamine comme l'éthyl-2 hexyl oxy-carbonyl n-méthyl glucamine, et leurs mélanges.

Comme alkylpolyglucosides, on utilise de préférence ceux contenant un groupe alkyle comportant de 6 à 30 atomes de carbone et de préférence de 8 à 16 atomes de carbone, et contenant un groupe hydrophile (glucoside) comprenant de préférence 1,2 à 3 unités de glucoside. Comme alkylpolyglucosides, on peut citer par exemple le decylglucoside (Alkyl-C9/C11-polyglucoside (1.4)) comme le produit commercialisé sous la dénomination MYDOL 10® par la société Kao Chemicals, le produit commercialisé sous la dénomination PLANTAREN 2000 UP® par la société Cognis, et le produit commercialisé sous la dénomination ORAMIX NS 10 ® par la société Seppic ; le caprylyl/capryl glucoside comme le produit commercialisé sous la dénomination ORAMIX CG 110® par la Société Seppic ; le laurylglucoside comme les produits commercialisés sous les dénominations PLANTAREN 1200 N® et PLANTACARE 1200® par la société Cognis ; le coco-glucoside comme le produit commercialisé sous la dénomination PLANTACARE 818/UP® par la société Cognis ; et leurs mélanges.

Les dérivés de maltose sont par exemple ceux décrits dans le document EP-A-566438, tels que l'O-octanoyl-6'-D-maltose, ou encore le O-dodecanoyl-6'-D-maltose décrit dans le document FR-A-2,739,556.

Comme alcools gras polyglycérolés, on peut citer par exemple le dodecanediol polyglycérolé (3,5 moles de glycérol), produit commercialisé sous la dénomination CHIMEXANE NF® par la société Chimex.

Les tensioactifs moussants amphotères et zwitterioniques peuvent être choisis par exemple parmi les dérivés de bétaïnes dont les amidopropylbétaïnes, les amphoacétates, les hydroxylsultaines et leurs mélanges.

Comme dérivés de bétaïnes, on peut citer par exemple la cocobétaïne comme le produit commercialisé sous la dénomination DEHYTON AB-30® par la société Cognis ; la laurylbétaïne comme le produit commercialisé sous la dénomination GENAGEN KB® par la société Clariant ; la laurylbétaïne oxyethylénée (10 OE), comme le produit commercialisé sous la dénomination LAURYLETHER(10 OE)BETAINE® par la société Shin Nihon Rica ; la stéarylbétaïne oxyéthylénée (10 OE) comme le produit commercialisé sous la dénomination STEARYLETHER(10 OE)BETAINE® par la société Shin Nihon Rica ; la cocamidopropyl betaine commercialisé par exemple sous la dénomination VELVETEX BK 35® par la société Cognis ; le undecylenamidopropyl betaïne commercialisé par exemple sous la dénomination AMPHORAM U® par la société Ceca ; et leurs mélanges.

Comme alkylamphoacétates, on peut citer par exemple le N-cocoyl-N-carboxyméthoxyéthyl-N-carboxyméthyl-éthylènediamine N-di-sodique (nom CTFA: disodium cocamphodiacetate) comme le produit commercialisé sous la dénomination MIRANOL C2M CONCENTRE NP® par la société Rhodia Chimie ; le N-cocoyl-N-hydroxyéthyl-N-carboxyméthyl-éthylènediamine N-sodique (nom CTFA : sodium cocamphoacetate) et leurs mélanges.

Les tensioactifs anioniques peuvent être choisis par exemple parmi les savons (sels alcalins d'acides gras), les dérivés d'aminoacides comme les acylaminoacide tels que les glycinates, les amidoéther carboxylates, les alkyl polyaminocarboxylates, les alkyl éthers sulfates tels que les Sodium Laureth sulfates, les alkyl sulfonates, les iséthionates, les alkyl méthyltaurate, les alkyl sulfosuccinates, les alkyl sulfoacétates, les alkylphosphates (monoalkylphosphates ou dialkylphosphates), leurs sels, et leurs mélanges.

Selon un mode préféré de réalisation de l'invention, les tensioactifs moussants autres que les acides hydroxy-2 alkyl carboxyliques sont choisis parmi les alkylpolyglucosides, les dérivés de bétaïnes, les alkylphosphates, les alkyl éthers sulfates et leurs mélanges.

### Système émulsionnant

Le système émulsionnant (B) contient un ou plusieurs émulsionnants. Les émulsionnants comme les tensioactifs moussants sont amphiphiles, c'est-à-dire qu'ils ont une partie hydrophile et une partie lipophile. Toutefois, ils n'ont pas de propriété moussante, mais ils ont la particularité de faciliter la dispersion de deux phases insolubles l'une dans l'autre, ce que ne font pas les tensioactifs moussants qui sont des détergents. La différence entre les émulsionnants et les tensioactifs moussants (ou détergents) tient à leur différence de HLB (Hydrophilic Lipophilic balance) et/ou leur différence de longueur de chaîne grasse. Le HLB est le rapport entre la partie hydrophile et la partie lipophile dans leur molécule. Ce terme HLB est bien connu de l'homme du métier et est décrit dans "The HLB system. A time-saving guide to Emulsifier Selection" (published by ICI Americas Inc ; 1984). Pour les émulsionnants le HLB va généralement de 3 à 8 pour la préparation des émulsions E/H et de 8 à 18 pour la préparation des émulsions H/E, alors que les tensioactifs moussants ont généralement un HLB supérieur à 20 et/ou une chaîne grasse comportant de 8 à 14 atomes de carbone.

Le système émulsionnant (B) utilisé dans la composition selon l'invention comprend un ou plusieurs émulsionnants dont la solubilité dans l'huile augmente avec l'augmentation de la température, émulsionnants permettant d'obtenir des émulsions par inversion de phase en température. Le HLB (hydrophilic lipophilic balance) du système émulsionnant va de 8 à 18 et de préférence de 10 à 16. Les émulsionnants du système émulsionnant peuvent être choisis notamment parmi les alcools gras éthoxylés, les acides gras éthoxylés, les glycérides partiels d'acides gras éthoxylés, les triglycérides d'acides gras polyglycérolés et leurs dérivés éthoxylés, et leurs mélanges.

Les émulsionnants sont de préférence choisis parmi les alcools gras éthoxylés ou les acides gras éthoxylés ayant les formules (I) et (II) suivantes :

R-O-(CH₂-CH₂-O)ₘH (I)

R-COO-(CH₂-CH₂-O)ₘH (II)

où R est une chaîne hydrocarbonée linéaire ou ramifiée, saturée ou insaturée, ayant de 10 à 24 atomes de carbone, et m est un nombre entier allant de 8 à 50.

Comme alcools gras éthoxylés, on peut citer par exemple les produits d'addition d'oxyde d'éthylène avec l'alcool laurylique, notamment ceux comportant de 9 à 50 groupes oxyéthylénés et plus particulièrement ceux comportant de 10 à 12 groupes oxyéthylénés (Laureth-10 à Laureth-12 en noms CTFA) ; les produits d'addition d'oxyde d'éthylène avec l'alcool béhénylique, notamment ceux comportant de 9 à 50 groupes oxyéthylénés (Beheneth-9 à Beheneth-50 en noms CTFA) ; les produits d'addition d'oxyde d'éthylène avec l'alcool cétéarylique (mélange d'alcool cétylique et d'alcool stéarylique), notamment ceux comportant de 10 à 30 groupes oxyéthylénés (Ceteareth-10 à Ceteareth-30 en noms CTFA) ; les produits d'addition d'oxyde d'éthylène avec l'alcool cétylique, notamment ceux comportant de 10 à 30 groupes oxyéthylénés (Ceteth-10 à Ceteth-30 en noms CTFA) ; les produits d'addition d'oxyde d'éthylène avec l'alcool stéarylique, notamment ceux comportant de 10 à 30 groupes oxyéthylénés (Steareth-10 à Steareth-30 en noms CTFA) ; les produits d'addition d'oxyde d'éthylène avec l'alcool isostéarylique, notamment ceux comportant de 10 à 50 groupes oxyéthylénés (Isosteareth-10 à Isosteareth-50 en noms CTFA) ; et leurs mélanges.

Comme acides gras éthoxylés, on peut citer par exemple les produits d'addition d'oxyde d'éthylène avec les acides laurique, palmitique, stéarique ou béhénique, et leurs mélanges, notamment ceux comportant de 9 à 50 groupes oxyéthylénés tels que les laurates de PEG-9 à PEG-50 (en noms CTFA : PEG-9 laurate à PEG-50 laurate) ; les palmitates de PEG-9 à PEG-50 (en noms CTFA : PEG-9 palmitate à PEG-50 palmitate) ; les stéarates de PEG-9 à PEG-50 (en noms CTFA : PEG-9 stearate à PEG-50 stearate) ; les palmito-stéarates de PEG-9 à PEG-50 ; les béhénates de PEG-9 à PEG-50 (en noms CTFA : PEG-9 behenate à PEG-50 behenate) ; et leurs mélanges.

On peut utiliser aussi des mélanges de ces dérivés oxyéthylénés d'alcools gras et d'acides gras.

Selon un mode préféré de réalisation de l'invention, le système émulsionnant de la composition de l'invention contient comme émulsionnant, au moins un alcool gras éthoxylé, et de préférence au moins l'alcool béhénylique.

Le système émulsionnant peut contenir éventuellement en outre un ou plusieurs co-émulsionnants. Comme co-émulsionnants, on peut citer par exemple les alcools gras ayant 12 à 30 atomes de carbone, comme par exemple l'alcool cétylique, l'alcool stéarylique, l'alcool béhénique ; les acides gras ayant 8 à 30 atomes de carbone, comme par exemple l'acide palmitique, l'acide stéarique, l'acide béhénique ; les esters gras de glycérol, comme par exemple le glycéryl stéarate ; les dérivés oxyéthylénés de ces alcools gras, acides gras et esters gras de glycérol, comportant 2 à 8 groupes oxyde d'éthylène, et leurs mélanges.

Le système émulsionnant (comprenant émulsionnants et co-émulsionnants) est généralement présent en une quantité allant de 2 à 20 %, de préférence de 3 à 16 % et mieux de 3 à 11 % en poids par rapport au poids total de la composition.

Selon un mode préféré de réalisation de l'invention, le rapport système émulsionnant (B) / phase huileuse (A) va de 0,04 à 0,2, de préférence de 0,06 à 0,18. Comme indiqué plus haut, on entend par « phase huileuse » l'ensemble des constituants qui ne sont pas hydrophiles et qui sont différents des émulsionnants ou co-émulsionnants du système émulsionnant.

### Phase huileuse

La phase huileuse appelée aussi phase lipophile ou grasse, est constituée des constituants lipophiles, c'est-à-dire huiles et autres corps lipophiles présents dans la composition, ainsi que de tous les additifs lipophiles éventuellement présents. La phase huileuse contient au moins une huile, notamment une huile cosmétique.

On entend par "huile ", un corps gras liquide à la température ambiante (20 à 25°C).

Selon un mode préféré de réalisation de l'invention, la phase huileuse de la composition selon l'invention comprend au moins une huile choisie parmi les huiles hydrocarbonées d'origine minérale ou synthétique ou alcanes, les esters gras, les éthers gras et leurs mélanges. On entend par «huile hydrocarbonée», toute huile comportant majoritairement des atomes de carbone et d'hydrogène, et éventuellement des groupements ester, éther, fluoré.
- Les huiles hydrocarbonées (alcanes) linéaires ou ramifiés, d'origine minérale, ou synthétique, peuvent être choisies par exemple parmi les huiles de paraffine, volatiles ou non, et leurs dérivés, la vaseline, l'huile de vaseline (Mineral oil), le perhydrosqualène, les polydécènes, l'isohexadecane, l'isododecane, le polyisobutène hydrogéné (ou isoparaffine hydrogénée) tel que l'huile de Parléam.
- Les esters gras peuvent être choisis par exemple parmi ceux obtenus à partir d'un alcool à chaîne linéaire, ou ramifiée, saturée ou insaturée ayant de 1 à 24 atomes de carbone et d'un acide gras à chaîne linéaire ou ramifiée, ayant de 3 à 24 atomes de carbone.
   Comme esters gras, on peut citer par exemple le caprate/caprylate d'éthyl-2 hexyle (ou caprate/caprylate d'octyle), le laurate d'éthyle, le laurate de butyle, le laurate d'hexyle, le laurate d'isohexyle, le laurate d'isopropyle, le myristate de méthyle, le myristate d'éthyle, le myristate de butyle, le myristate d'isobutyle, le myristate d'isopropyle, le myristate d'octyl-2 dodécyle, le monococoate d'éthyl-2 hexyle (ou monococoate d'octyle), le palmitate de méthyle, le palmitate d'éthyle, le palmitate d'isopropyle, le palmitate d'isobutyle, palmitate d'ethyl-2 hexyle (ou palmitate d'octyle), le stéarate de butyle, le stéarate d'isopropyle, le stéarate d'isobutyle, le stéarate d'éthyl-2 hexyle (ou stéarate d'octyle), l'isostéarate d'isopropyle, le stéarate d'isocétyle, l'isostéarate d'isotéaryle, le pelargonate d'ethyl-2 hexyle (ou pelargonate d'octyle), l'hydroxystéarate d'éthyl-2 hexyle (ou hydroxystéarate d'octyle), le decyl oleate, l'adipate de di-isopropyle, l'adipate de di-éthyl-2 hexyle (ou adipate de di-octyle), l'adipate de diisocetyle, le succinate d'ethyl-2 hexyle (ou succinate d'octyle), le sébacate de diisopropyle, le malate d'ethyl-2 hexyle (ou malate d'octyle), le caprate/caprylate de pentaérythritol, l'hexanoate d'éthyl-2 hexyle (ou hexanoate d'octyle), l'octanoate d'octyldodécyle, le néopentanoate d'isodécyle, le néopentanoate d'isostéaryle, le neopentanoate d'octyldodecyle, l'isononanoate d'isononyle, l'isononanoate d'isotridécyle, l'isononanoate de cétéaryle, l'isononanoate d'isodécyle, l'isononanoate d'isotridécyle, le lactate de lauryle, le lactate de myristyle, le lactate de cétyle, le propionate de myristyle, l'éthyl-2 hexanoate d'éthyl-2 hexyle (ou éthyl-2 hexanoate d'octyle), l'octanoate d'éthyl-2 hexyle (ou octanoate d'octyle), l'ethyl-2 hexanoate de cetyle, le pentaérythritol de tétraisostéarate, le lauroyl sarcosinate d'isopropyle (Eldew SL 205 de la société Unipex), le dicaprylyl carbonate (Cetiol CC de la société Cognis), le benzoates d'alcools gras en C12-C15 (Finsolv TN de la société FINETEX).
- Comme éthers gras, on peut citer le Dicaprylyl ether (Cetiol OE de la société Cognis).

On peut aussi utiliser dans la phase huileuse (A), les huiles suivantes :
- les huiles hydrocarbonées d'origine végétale telles que l'huile d'amande douce, l'huile d'avocat, l'huile de ricin, l'huile de coriandre, l'huile d'olive, l'huile de jojoba, l'huile de sésame, l'huile d'arachide, l'huile de pépins de raisin, l'huile de colza, l'huile de coprah, l'huile de noisette, le beurre de karité, l'huile de palme, l'huile de noyau d'abricot, l'huile de calophyllum, l'huile de son de riz, l'huile de germes de maïs, l'huile de germes de blé, l'huile de soja, l'huile de tournesol, l'huile d'onagre, l'huile de carthame, l'huile de passiflore, l'huile de seigle, les triglycérides des acides caprylique/caprique comme ceux vendus par la société Stearineries Dubois ou ceux vendus sous les dénominations Miglyol 810, 812 et 818 par la société Dynamit Nobel ;
- les huiles de silicone, volatiles ou non, sous réserve qu'elles ne nuisent pas à la qualité moussante, comme les polydiméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, les polyméthyl-phénylsiloxanes ;
- les huiles fluorées, sous réserve qu'elles ne nuisent pas à la qualité moussante, telles que celles partiellement hydrocarbonées et/ou siliconées comme celles décrites dans le document JP-A-2-295912.

Pour obtenir une meilleure qualité de mousse, il est avantageux que la phase huileuse de la composition de l'invention contienne une ou plusieurs huiles ayant un poids moléculaire supérieur ou égal à 360 g/mole, choisies notamment parmi les alcanes et les esters d'acide gras de poids moléculaire supérieur ou égal à 360 g/mole.

Comme alcanes, on utilise de préférence la vaseline, l'huile de vaseline, l'isoparaffine hydrogénée (ou polyisobutène hydrogéné) tel que l'huile de Parléam. L'huile de vaseline présente l'avantage de donner une mousse couvrante et un bon démarrage de mousse.

Comme esters gras de poids moléculaire supérieur ou égal à 360 g/mole, on peut citer par exemple le palmitate d'ethyl-2 hexyle (ou palmitate d'octyle), l'ethyl-2 hexanoate de cetyle, le myristate d'octyl-2 dodécyle, le stéarate d'isocétyle, l'isostéarate d'isotéaryle, le stéarate d'éthyl-2 hexyle (ou stéarate d'octyle), le neopentanoate d'octyldodecyle, l'isononanoate de cétéaryle, l'isononanoate d'isodécyle, le pentaérythritol de tétraisostéarate, l'isononanoate d'isotridécyle.

De préférence, la composition selon l'invention contient un ou plusieurs esters gras choisis parmi ceux cités ci-dessus. Plus préférentiellement, la phase huileuse de la composition de l'invention contient au moins une huile choisie parmi l'huile de vaseline (Mineral oil), le palmitate d'ethyl-2 hexyle (ou palmitate d'octyle), l'éthyl-2 hexanoate de cétyle (ou octanoate de cétyle) et leurs mélanges.

La phase huileuse peut comprendre des additifs lipophiles.

La phase huileuse est présente en une quantité d'au moins 30 % en poids, de préférence de plus de 30 % en poids et mieux d'au moins 35 % en poids par rapport au poids total de la composition. La quantité de phase huileuse peut aller par exemple de 32 à 70 % en poids, de préférence de 35 à 70 %, mieux de 35 à 60 % en poids et mieux de 40 à 60 % en poids par rapport au poids total de la composition.

### Phase aqueuse

La phase aqueuse comprend l'eau, les tensioactifs moussants et les composés hydrophiles éventuellement présents. La composition selon l'invention comprend généralement une quantité d'eau inférieure ou égale à 45 % et de préférence inférieure ou égale à 30 % du poids total de la composition, cette quantité pouvant aller par exemple de 10 à 45 % en poids et de préférence de 10 à 30 % en poids par rapport au poids total de la composition.

Selon un mode préféré de réalisation de l'invention, la composition de l'invention contient au moins un polyol (ou alcools polyhydriques) qui est généralement présent dans la phase aqueuse. Comme polyols, on peut citer par exemple la glycérine ; les glycols comme le propylène glycol, le butylène glycol, l'isoprène glycol et les polyéthylène glycols tels que le PEG-8 ; le sorbitol ; les sucres tels que le glucose, le fructose, le maltose, le lactose, le sucrose ; et leurs mélanges. Lorsqu'ils sont présents, la quantité de polyol(s) dans la composition de l'invention peut aller par exemple de 0,01 à 30 % en poids, de préférence de 2 à 20 % en poids et mieux de 5 à 15 % en poids par rapport au poids total de la composition.

De manière classique, la phase aqueuse peut contenir, outre l'eau et le ou les polyols, un ou plusieurs solvants hydrosolubles choisis parmi les alcool(s) inférieur(s) hydrosoluble(s). On entend par alcool inférieur, un alcool comportant de 1 à 8 atomes de carbone. Comme alcools inférieurs, on peut citer par exemple l'éthanol, l'isopropanol, le butanol et leurs mélanges. Lorsqu'ils sont présents dans la composition de l'invention, le ou les alcool(s) inférieur(s) hydrosoluble(s) peuvent être en une quantité allant de 0,01 à 20 % en poids et de préférence de 0,1 à 10 % en poids par rapport au poids total de la composition, sous réserve qu'ils ne nuisent ni à la qualité moussante ni à la stabilité de la composition.

### Additifs

La composition selon l'invention peut aussi contenir tout adjuvant ou additif habituellement utilisé dans les domaines considérés et notamment dans les domaines cosmétique ou dermatologique. Bien entendu, l'homme de l'art veillera à choisir le ou les éventuels additifs de la composition selon l'invention de manière telle que les propriétés avantageuses attachées intrinsèquement à la composition conforme à l'invention ne soient pas ou substantiellement pas, altérées par l'addition envisagée.

Parmi les adjuvants classiques susceptibles d'être contenus dans la phase aqueuse et/ou dans la phase huileuse des émulsions conformes à l'invention (selon le caractère hydrosoluble ou liposoluble de ces adjuvants), on peut citer les conservateurs ; les séquestrants (EDTA) ; les antioxydants ; les parfums ; les matières colorantes telles que les colorants solubles, les pigments et les nacres ; les charges à effets matifiant, tenseur, blanchissant ou exfoliant ; les filtres solaires ; les actifs cosmétiques ou dermatologiques, hydrophiles ou lipophiles, tels que les vitamines, les antiseptiques, les antisébohrréïques, les antimicrobiens comme le peroxyde de benzoyle, l'acide salicylique, le triclosan, l'acide azélaïque, la niacine (vit. PP), les amincissants comme la caféine, les azurants optiques, les électrolytes, les agents ayant pour effet d'améliorer les propriétés cosmétiques de la peau ou encore des particules solides sphériques ou non, poreuses ou non de toute taille. Les quantités de ces différents adjuvants sont celles classiquement utilisées dans le domaine considéré, et par exemple de 0,01 à 20 % du poids total de la composition. Ces adjuvants ainsi que leurs concentrations doivent être tels qu'ils ne modifient pas la propriété recherchée pour la composition de l'invention.

Comme charges, on peut citer notamment la silice.

Les compositions peuvent contenir également des polymères hydrophiles ou lipophiles, anioniques, non ioniques, cationiques ou amphotères, épaississants ou dispersants, dans la mesure où ils n'affectent pas les qualités de l'émulsion ni de la mousse.

Les émulsions selon l'invention sont de préférence obtenues par un procédé d'inversion de phase, tel que décrit ci-dessus.

De manière plus particulière, le procédé de préparation consiste à :
- 1) Peser dans un récipient tous les constituants de la composition (à l'exception des tensioactifs moussants, et, s'il y en a, des charges et des matières premières thermosensibles)
- 2) Homogénéiser le mélange, par exemple au moyen d'un Rayneri 350 tours/min, tout en chauffant en augmentant progressivement la température au moyen d'un bain marie jusqu'à une température supérieure ou égale à la température d'inversion de phase T2, c'est-à-dire jusqu'à l'obtention d'une phase transparente ou translucide (zone de microémulsion ou de phase lamellaire) puis d'une phase blanche plus visqueuse qui indique l'obtention de l'émulsion inverse (E/H).
- 3) Arrêter le chauffage et maintenir l'agitation jusqu'à retour à la température ambiante, en passant par la température d'inversion de phase T1, c'est-à-dire la température à laquelle se forme une émulsion H/E fine.
- 4) Lorsque la température est redescendue en dessous de la zone d'inversion de Phase en Température (T1), additionner éventuellement les particules (exemple silice).
- 5)Vers 45°C , ajouter les tensioactifs moussants.
- 6) Vers 30°C, ajouter les matières premières thermosensibles éventuellement présentes.

La température T1 correspond à la formation d'une microémulsion bicontinue ; le mélange est translucide homogène ; et la température T2 correspond à la formation d'une émulsion eau dans huile : le mélange devient opaque blanc et s'épaissit.

On obtient une émulsion H/E stable dont les gouttelettes d'huile sont fines.

Dans la zone de formation d'une microémulsion (mélange translucide de micelles), les interactions hydrophiles et hydrophobes sont équilibrées car la tendance du tensioactif est de former aussi bien des micelles directes que des micelles inverses. Par chauffage au-delà de cette zone, il y a formation d'une émulsion E/H (mélange opaque blanc), car le tensioactif favorise la formation d'une émulsion eau dans huile. Puis lors du refroidissement en dessous de la zone d'inversion de phase, l'émulsion devient une émulsion H/E.

Les compositions selon l'invention se présentent sous forme de crèmes plus ou moins souples, et elles peuvent constituer notamment des compositions cosmétiques ou dermatologiques. Elles peuvent être utilisées sur toutes les matières kératiniques telles que la peau, le cuir chevelu, les cheveux, les cils, les sourcils, les ongles ou les muqueuses, notamment comme produit d'hygiène, par exemple comme produit de nettoyage de la peau, des muqueuses et/ou des cheveux, en particulier comme produit de nettoyage et/ou de démaquillage de la peau (du visage et/ou du corps), comme produit de douche (produit deux-en-un), comme shampoing ou après-shampoing, comme produit de rasage, comme masque à rincer, comme produit exfoliant (appelé aussi desquamant ou désincrustant) aussi bien pour le visage que pour le corps ou pour les mains, après addition de particules exfoliantes.

L'invention a encore pour objet l'utilisation cosmétique de la composition telle que définie ci-dessus, comme produit de nettoyage et/ou de démaquillage de la peau, comme produit de douche, comme shampoing, comme après-shampoing, comme produit de rasage, comme masque à rincer, comme produit exfoliant.

Un autre objet de l'invention est un procédé de nettoyage d'une matière kératinique, telle que la peau, du cuir chevelu, des cheveux, des cils, des sourcils, des ongles ou des muqueuses, caractérisé par le fait qu'on applique sur la matière kératinique, une composition telle que définie ci-dessus, et qu'on la rince.

La matière kératinique est de préférence la peau.

Les compositions selon l'invention sont rincées après application.

Les compositions selon l'invention peuvent être par exemple utilisées de la manière suivante :
1) quand les compositions selon l'invention constituent des produits de nettoyage doux pour le visage, elles peuvent être utilisées de la manière suivante :
   - on fait mousser le produit dans les mains avec de l'eau
   - on applique la mousse sur le visage
   - on nettoie le visage
   - on rince à l'eau
      Résultat : la peau est nettoyée sans être agressée.
2) quand les compositions selon l'invention constituent des produits démaquillants, elles peuvent être utilisées comme indiqué ci-dessus mais elles peuvent aussi être appliquées à sec sur le visage, puis massées jusqu'à obtention d'un démaquillage satisfaisant, et rincées à l'eau.
   Résultat : la peau est démaquillée sans laisser de résidu gras désagréable.
3) on peut les utiliser comme produit de douche deux-en-un de la manière habituelle d'utilisation des produits de douche.
4) on peut les utiliser comme shampooing et/ou après shampooing, comme produit de rasage ou comme masque à rincer de la manière habituelle d'utilisation de ces produits.
5) on peut les utiliser comme produit désincrustant aussi bien pour le visage que pour les mains (lorsque la composition contient des particules exfoliantes) L'utilisation consiste à appliquer le produit sur le visage ou les mains ou le corps, à frotter une minute ou deux puis à rincer. La peau est alors lisse, douce et désincrustée.

Les exemples décrits ci-dessous ont été soumis à des mesures de viscosité permettant de mettre en évidence leur stabilité dans le temps, et à des tests permettant de tester leurs qualités de mousse.

### Performances de mousse :

Pour certains des exemples, les performances de mousse des compositions (qualités de mousse) ont été déterminées selon le protocole décrit ci-dessous.

Avant toute utilisation du produit, les mains sont lavées au savon de Marseille puis convenablement rincées et séchées. Puis le protocole suivi est le suivant :
1- mouiller les mains en les passant sous l'eau courante, les secouer trois fois pour les enlever l'excès d'eau,
2- placer 1 g de produit dans le creux de l'une des mains,
3- travailler le produit entre les deux paumes pendant 10 secondes,
4- ajouter 2 ml d'eau et travailler le produit à nouveau pendant 10 secondes,
5- rincer les mains sous l'eau,
6- les essuyer.

Les critères sont évalués à chaque étape du protocole suivi, et ils sont notés sur une échelle de 0 à 10. On considère que, pour un critère donné, il y a une différence de note entre deux produits lorsque la différence entre 2 notes est supérieure ou égale à 1.
- étape 3 : évaluation du pouvoir couvrant : la note attribuée est d'autant plus élevée que la peau de la main ne se voit pas à travers le produit étalé sur celle ci.
- étapes 4 et 5 : évaluation de la qualité de mousse
   - Le volume de mousse : la note attribuée est d'autant plus élevée que le volume est grand.
   - La taille des bulles composant la mousse : la note attribuée est d'autant plus élevée que les bulles sont grosses.
   - La densité : consistance, tenue de la mousse ; la note attribuée est d'autant plus élevée que la densité est grande.
   - La douceur de la mousse : la note attribuée est d'autant plus élevée que la mousse est douce.
- étape 6 : évaluation pendant le rinçage :Le rinçage : la note attribuée est d'autant plus faible que la présence d'un film glissant difficile à éliminer est grande.

Le panel d'évaluation est constitué de 4 experts entraînés. La moyenne des 4 notes permet de comparer les compositions selon chacun des critères.

Les exemples indiqués ci-dessous permettront de mieux comprendre l'invention sans toutefois présenter un caractère limitatif. Dans ces exemples, les quantités indiquées sont en % en poids de matière active (et non en quantité de matière première). Les composés sont cités en nom chimique ou en nom CTFA.

Les matières premières utilisées dans les exemples ont été les suivantes, leurs quantités ayant été adaptées à la quantité de matière active des exemples :
(1) EUMULGIN BA 10® commercialisé par la société Cognis = Alcool béhénylique oxyéthyléné (10 OE).
(2) ETHYL HEXYL STEARATE® commercialisé par la société Cognis = stéarate d'octyle.
(3) BEAULIGHT SHAA® commercialisé par la société Sanyo = solution de Sodium lauryl glycol carboxylate à une concentration en matière active de 29% en poids.
(4) ORAMIX CG 110® commercialisé par la société Seppic = solution de Decyl glucoside à une concentration en matière active de 60% en poids.
(5) Aerosil 200® commercialisé par la société Degussa)
(6) GENAPOL LRO B® = solution aqueuse de Sodium Laureth Sulfate, commercialisé par CLARIANT à une concentration en matière active de 70% en poids.
(7) PALMITATE D'ETHYL 2 HEXYLE® commercialisé par la société Stéarinerie Dubois = palmitate d'octyle.
(8) Genagen KB® commercialisé par la société Clariant ou Mirataine BB/FLA® commercialisé par la société Rhodia ou Empigen BB/LS® commercialisé par la société Huntsman ou Dehyton AB 30® commercialisé par la société Cognis : solution de Cocobétaïne à une concentration en matière active de 30% en poids.
(9) AKYPO RLM 45 CA de la société KAO

### Exemple 1 selon l'invention

| Compositions | Exemple 1 selon l'invention |
|---|---|
| Beheneth-10 (1) | 4 |
| Stéarate d'octyle (2) | 50,4 |
| Sodium lauryl glycol carboxylate (3) | 5,8 |
| Decyl glucoside (4) | 3 |
| Propyl paraben | 0,15 |
| Methyl paraben | 0,15 |
| Chlorhexidine digluconate | 0,25 |
| Glycérine | 6,5 |
| Silice (5) | 2 |
| Ethanol | 2 |
| Sodium citrate | 0.025 (qs pH 6,9) |
| Eau permutée | Qsp100 % |
| Aspect | Crème blanche onctueuse qui ne coule pas. |
| Viscosité (Pa.s) après 24 heures à température ambiante (25°C) après 10 minutes de rotation du mobile. | 2,5 Pa.s |

### Tableau sur les qualités de mousse

| | Exemple 1 selon l'invention |
|---|---|
| Démarrage | 7,5 |
| Mélange à l'eau | 10,0 |
| Homogénéité | 10,0 |
| Couvrant | 6,3 |
| Volume mousse + 2ml d'eau (étape 4) | 3,4 |
| Taille bulles+ 2ml d'eau (étape 4) | 3,6 |
| Densité + 2ml d'eau (étape 4) | 4,4 |
| Volume mousse + 4ml d'eau (étape 5) | 5,0 |
| Taille bulles+ 4ml d'eau (étape 5) | 4,1 |
| Densité+ 4ml d'eau (étape 5) | 4,1 |
| Douceur | 6,5 |
| Rinçage | 7,6 |

Ces résultats montrent que la composition selon l'invention donne une mousse qui démarre bien, qui est homogène, a une bonne densité et se rince bien.

### Exemple 2 selon l'invention et exemple comparatif 1

| Composition | Exemple comparatif 1 | Exemple 2 de l'invention |
|---|---|---|
| Beheneth-10 (1) | 4 | 4 |
| Stéarate d'octyle (2) | 50,4 | 50,4 |
| Sodium lauryl glycol carboxylate (3) | - | 5,8 |
| Decyl glucoside (4) | 3 | 3 |
| Sodium Laureth Sulfate (6) | 5,8 | - |
| Propyl paraben | 0,15 | 0,15 |
| Methyl paraben | 0,15 | 0,15 |
| Chlorhexidine digluconate | 0,25 | 0,25 |
| glycérine | 6,5 | 6,5 |
| Silice (5) | 2 | 2 |
| Ethanol | 2 | 2 |
| Sodium citrate | 0.025 (qs pH 6,9) | 0.025 (qs pH 6,9) |
| Eau permutée | Qsp 100 % | Qsp100 % |
| Aspect | Crème blanche onctueuse qui ne coule pas. | Crème blanche onctueuse qui ne coule pas. |
| pH | 6,9 | 6,9 |
| Viscosité (Pa.s) après 24 heures à température ambiante (25°C); après 10 minutes de rotation du mobile M3 | 3,7 | 6 |

La comparaison des qualités moussantes dans le tableau ci-dessous montre que la composition contenant le Beaulight Shaa présente des meilleures performances pour le démarrage en mousse, l'effet couvrant, le volume et la densité de mousse par rapport à l'exemple comparatif contenant un tensioactif classique tel que le lauryl ether sulfate de sodium.

### Tableau sur les qualités de mousse

| | Exemple comparatif 1 | Exemple 2 selon l'invention |
|---|---|---|
| Démarrage | 2.5 | 7,5 |
| Mélange à l'eau | 10 | 10,0 |
| Homogénéité | 10 | 10,0 |
| Couvrant | 3 | 6,3 |
| Volume mousse + 2ml | 2 | 3,4 |
| Taille bulles+ 2ml | 3,5 | 3,6 |
| Densité + 2ml | 2,5 | 4,4 |
| Volume mousse + 4ml | 3 | 5,0 |
| Taille bulles+ 4ml | 4,6 | 4,1 |
| Densité+ 4ml | 1,3 | 4,1 |
| Douceur | 6,5 | 6,5 |
| Rinçage | 6 | 7,6 |

### Exemples 3 et 4 selon l'invention

| Composition | Exemple 3 (démaquillant moussant) | Exemple 4 (moussant) |
|---|---|---|
| Beheneth-10 (1) | 4 | 4 |
| Palmitate d'octyle (7) | 40 | - |
| Stéarate d'octyle (2) | - | 50.4 |
| PEG-6 Capric/Caprylic Glycerides | 1,55 | - |
| Sodium lauryl glycol carboxylate (3) | 2,9 | 3,8 |
| Decyl glucoside (4) | 7,2 | 3 |
| Cocobétaïne (8) | 0,9 | 2,1 |
| Silice (5) | 2 | 2 |
| Isononanoate d'isononyle | 12.4 | - |
| Propyl paraben | 0,15 | 0,15 |
| Methyl paraben | 0,15 | 0,15 |
| Chlorhexidine digluconate | 0.25 | - |
| glycérine | 6,5 | 6,5 |
| Ethanol | 2 | - |
| Eau permutée | Qsp100 % | Qsp100 % |
| Aspect | Crème blanche onctueuse qui ne coule pas. | Crème blanche onctueuse qui ne coule pas. |
| Viscosité (Pa.s) à 24 h t amb.; 25°C; après t10 minutes de rotation du mobile M3. | 3,4 | 6 |
| Pouvoir démaquillant | 73% | Non mesuré |

### Exemples 5 selon l'invention et exemple comparatif 2

| Composition | Exemple5 (démaquillant moussant) | Exemple comparatif 2 |
|---|---|---|
| Behenth-10 (1) | 4 | 4 |
| Palmitate d'octyle (8) | 30.4 | 30.4 |
| Huile de Vaseline (Mineral oil) | 20 | 20 |
| Sodium lauryl glycol carboxylate (3) | 20 | - |
| Laureth-5 carboxylique acid (4) | | 20 |
| Propyl paraben | 0.15 | 0.15 |
| Methyl paraben | 0.15 | 0.15 |
| Silice (AEROSIL 200® de Degussa) | 2 | 2 |
| Isononanoate d'isononyle | 12.4 | 12.4 |
| Propyl paraben | 0.15 | 0.15 |
| glycérine | 6,5 | 6.5 |
| Hexametaphosphate de sodium | 2 | 2 |
| Ethanol | 2 | 2 |
| Eau permutée | Qsp100 % | Qsp 100 % |
| | | |
| Aspect | Crème blanche onctueuse qui ne coule pas. | Composition fluide et déphasée |
| Viscosité (Pa.s) à 24 h à température ambinate ( 25 °C) après 10 minutes de rotation du mobile M3 . | 3,6 | Impossible à mesurer car produit déphasé |
| pH | 6.0 | Impossible à mesurer car produit déphasé |
| Propriétés | mousse bien | Ne mousse pas |

L'exemple 2 comparatif montre que le but de l'invention n'est pas atteint en utilisant un tensioactif carboxylique autre que ceux de formule (I).

## Revendications

1. Composition moussante pour application topique sous forme d'émulsion huile-dans-eau, comprenant une phase huileuse dispersée dans une phase aqueuse, **caractérisée en ce que** :
- la taille des gouttelettes de la phase huileuse D[4,3] est inférieure ou égale à 4 µm,
- la phase huileuse (A) est présente en une quantité d'au moins 30 % en poids par rapport au poids total de la composition,
et **en ce que** la composition contient :
- un système émulsionnant (B) ayant un HLB allant de 8 à 18, présent en une quantité d'au moins 2 % en poids par rapport au poids total de la composition ,
- un système moussant (C) comprenant au moins un tensioactif anionique choisi parmi les acides alkyl glycol carboxyliques et leurs sels, de formule (I) suivante :
R₁-CHOH-CH₂-O-CH₂-COO⁻ X⁺ (I)
dans laquelle R₁ désigne un radical alkyle saturé ou insaturé, linéaire ou ramifié, comportant de 8 à 30 atomes de carbone, et X désigne l'hydrogène ou un cation minéral ou organique,
- le rapport en poids phase huileuse (A) / système moussant (C) allant de 1,5 à 12.

2. Composition selon la revendication 1, **caractérisée en ce qu'**elle est susceptible d'être obtenue selon la technique d'émulsification par inversion de phase.

3. Composition selon la revendication 1 ou 2, **caractérisée en ce que** la taille moyenne des gouttelettes de phase huileuse D[4,3] va de 0,09 µm à 4 µm.

4. Composition selon l'une quelconque des revendications précédentes, **caractérisée en ce que** le tensioactif anionique est un composé de formule (I) dans laquelle R₁ désigne un radical alkyle saturé ou insaturé, linéaire ou ramifié comportant de 8 à 18 atomes de carbone.

5. Composition selon la revendication précédente, **caractérisée en ce que** le tensioactif anionique choisi parmi les acides alkyl glycol carboxyliques et leurs sels est le lauryl glycol carboxylate de sodium, ou sa forme acide.

6. Composition selon l'une quelconque des revendications précédentes, **caractérisée en ce que** le système moussant contient en outre un ou plusieurs tensioactifs moussants choisis parmi les alkylpolyglucosides, les dérivés de bétaïnes, les alkylphosphates, les alkyl éthers sulfates et leurs mélanges.

7. Composition selon l'une quelconque des revendications précédentes, **caractérisée en ce que** la quantité de système moussant va de 0,5 à 20 % en poids par rapport au poids total de la composition.

8. Composition selon l'une quelconque des revendications précédentes, **caractérisée en ce que** le rapport en poids phase huileuse (A) / système moussant (C) va de 4 à 10.

9. Composition selon l'une quelconque des revendications précédentes, **caractérisée en ce que** l'émulsionnant est choisi parmi les alcools gras éthoxylés, les acides gras éthoxylés, les glycérides partiels d'acides gras ethoxylés, les triglycérides d'acides gras polyglycérolés et leurs dérivés éthoxylés, et leurs mélanges.

10. Composition selon la revendication précédente, **caractérisée en ce que** l'émulsionnant est choisi parmi les produits d'addition d'oxyde d'éthylène avec l'alcool laurylique ; les produits d'addition d'oxyde d'éthylène avec l'alcool béhénylique ; les produits d'addition d'oxyde d'éthylène avec l'alcool cétéarylique ; les produits d'addition d'oxyde d'éthylène avec l'alcool cétylique ; les produits d'addition d'oxyde d'éthylène avec l'alcool stéarylique ; les produits d'addition d'oxyde d'éthylène avec l'alcool isostéarylique ; les produits d'addition d'oxyde d'éthylène avec les acides laurique, palmitique, stéarique ou béhénique, et leurs mélanges.

11. Composition selon l'une quelconque des revendications précédentes, **caractérisée en ce que** le système émulsionnant contient en outre un ou plusieurs co-émulsionnants choisis parmi les alcools gras ayant 8 à 30 atomes de carbone ; les acides gras ayant 8 à 30 atomes de carbone ; les esters gras de glycérol ; leurs dérivés oxyéthylénés comportant 2 à 8 groupes oxyde d'éthylène, et leurs mélanges.

12. Composition selon l'une quelconque des revendications précédentes, **caractérisée en ce que** la quantité de système émulsionnant va de 2 à 20 % en poids par rapport au poids total de la composition.

13. Composition selon l'une quelconque des revendications précédentes, **caractérisée en ce que** le rapport système émulsionnant (B) / phase huileuse (A) va de 0,04 à 0,2.

14. Composition selon l'une quelconque des revendications précédentes, **caractérisée en ce que** la quantité de phase huileuse va de 30 à 70 % en poids par rapport au poids total de la composition.

15. Composition selon l'une quelconque des revendications précédentes, **caractérisée en ce que** la phase huileuse contient une ou plusieurs huiles ayant un poids moléculaire supérieur ou égal à 360 g/mole.

16. Composition selon la revendication précédente, **caractérisée en ce que** la phase huileuse contient au moins une huile choisie parmi l'huile de vaseline, le palmitate d'ethyl-2 hexyle, l'éthyl-2 hexanoate de cétyle et leurs mélanges.

17. Composition selon l'une quelconque des revendications précédentes, **caractérisée en ce qu'**elle comprend une quantité d'eau inférieure ou égale à 45 % du poids total de la composition.

18. Composition selon l'une quelconque des revendications précédentes, **caractérisée en ce qu'**elle constitue un produit de nettoyage et/ou de démaquillage de la peau, un produit de douche, un shampoing ou un après-shampoing, un produit de rasage, un masque à rincer, un produit exfoliant.

19. Utilisation cosmétique d'une composition cosmétique selon l'une quelconque des revendications 1 à 17, comme produit de nettoyage et/ou de démaquillage de la peau, comme produit de douche, comme shampoing, comme après-shampoing, comme produit de rasage, comme masque à rincer, comme produit exfoliant.

20. Procédé de nettoyage d'une matière kératinique, **caractérisé par le fait qu'**on applique sur la matière kératinique, une composition selon l'une quelconque des revendications 1 à 17, et qu'on la rince.

## Claims

1. Foaming composition for topical application in the form of an oil-in-water emulsion, comprising an oily phase dispersed in an aqueous phase, **characterized in that**:
- the size of the droplets of the oily phase D[4,3] is less than or equal to 4 µm,
- the oily phase (A) is present in an amount of at least 30% by weight relative to the total weight of the composition,
and **in that** the composition contains:
- an emulsifying system (B) with an HLB value ranging from 8 to 18, present in an amount of at least 2% by weight relative to the total weight of the composition,
- a foaming system (C) comprising at least one anionic surfactant chosen from alkyl glycol carboxylic acids and salts thereof, of formula (I) below:
R₁-CHOH-CH₂-O-CH₂COO⁻X⁺ (I)
in which R₁ denotes a saturated or unsaturated, linear or branched alkyl radical containing from 8 to 30 carbon atoms, and X denotes hydrogen or a mineral or organic cation,
- the oily phase (A)/foaming system (C) weight ratio ranging from 1.5 to 12.

2. Composition according to Claim 1, **characterized in that** it may be obtained according to the phase inversion emulsification technique.

3. Composition according to Claim 1 or 2, **characterized in that** the mean size of the droplets of oily phase D[4,3] ranges from 0.09 µm to 4 µm.

4. Composition according to any one of the preceding claims, **characterized in that** the anionic surfactant is a compound of formula (I) in which R₁ denotes a saturated or unsaturated, linear or branched alkyl radical containing from 8 to 18 carbon atoms.

5. Composition according to the preceding claim, **characterized in that** the anionic surfactant chosen from alkyl glycol carboxylic acids and salts thereof is sodium lauryl glycol carboxylate, or its acid form.

6. Composition according to any one of the preceding claims, **characterized in that** the foaming system also contains one or more foaming surfactants chosen from alkyl polyglucosides, betaine derivatives, alkyl phosphates and alkyl ether sulfates, and mixtures thereof.

7. Composition according to any one of the preceding claims, **characterized in that** the amount of foaming system ranges from 0.5% to 20% by weight relative to the total weight of the composition.

8. Composition according to any one of the preceding claims, **characterized in that** the oily phase (A)/foaming system (C) weight ratio ranges from 4 to 10.

9. Composition according to any one of the preceding claims, **characterized in that** the emulsifier is chosen from ethoxylated fatty alcohols, ethoxylated fatty acids, partial glycerides of ethoxylated fatty acids, triglycerides of polyglycerolated fatty acids and ethoxylated derivatives thereof, and mixtures thereof.

10. Composition according to the preceding claim, **characterized in that** the emulsifier is chosen from the products of addition of ethylene oxide with lauryl alcohol; the products of addition of ethylene oxide with behenyl alcohol; the products of addition of ethylene oxide with cetearyl alcohol; the products of addition of ethylene oxide with cetyl alcohol; the products of addition of ethylene oxide with stearyl alcohol; the products of addition of ethylene oxide with isostearyl alcohol; the products of addition of ethylene oxide with lauric acid, palmitic acid, stearic acid or behenic acid, and mixtures thereof.

11. Composition according to any one of the preceding claims, **characterized in that** the emulsifying system also contains one or more coemulsifiers chosen from fatty alcohols containing 8 to 30 carbon atoms; fatty acids containing 8 to 30 carbon atoms; fatty esters of glycerol; oxyethylenated derivatives thereof containing 2 to 8 ethylene oxide groups, and mixtures thereof.

12. Composition according to any one of the preceding claims, **characterized in that** the amount of emulsifying system ranges from 2% to 20% by weight relative to the total weight of the composition.

13. Composition according to any one of the preceding claims, **characterized in that** the emulsifying system (B)/oily phase (A) ratio ranges from 0.04 to 0.2.

14. Composition according to any one of the preceding claims, **characterized in that** the amount of oily phase ranges from 30% to 70% by weight relative to the total weight of the composition.

15. Composition according to any one of the preceding claims, **characterized in that** the oily phase contains one or more oils with a molecular weight of greater than or equal to 360 g/mol.

16. Composition according to the preceding claim, **characterized in that** the oily phase contains at least one oil chosen from liquid petroleum jelly, 2-ethylhexyl palmitate and cetyl 2-ethylhexanoate, and mixtures thereof.

17. Composition according to any one of the preceding claims, **characterized in that** it comprises an amount of water of less than or equal to 45% of the total weight of the composition.

18. Composition according to any one of the preceding claims, **characterized in that** it is a skin cleansing and/or makeup-removing product, a shower product, a shampoo or a conditioner, a shaving product, a rinse-off mask or an exfoliant product.

19. Cosmetic use of a cosmetic composition according to any one of Claims 1 to 17, as a skin cleansing and/or makeup-removing product, as a shower product, as a shampoo, as a conditioner, as a shaving product, as a rinse-off mask or as an exfoliant product.

20. Process for cleansing a keratin material, **characterized in that** a composition according to any one of Claims 1 to 17 is applied to the keratin material, and is rinsed off.

## Patentansprüche

1. Schaumbildende Zusammensetzung für die topische Anwendung in Form einer Öl-in-Wasser-Emulsion, die eine in einer wässrigen Phase dispergierte Ölphase aufweist, **dadurch gekennzeichnet, dass**:
- die Tröpfchengröße der Ölphase D[4,3] höchstens 4 µm beträgt,
- die Ölphase (A) in einer Menge von mindestens 30 Gew.-%, bezogen auf das Gesamtgewicht der Zusammensetzung, enthalten ist,
und dadurch, dass die Zusammensetzung enthält:
- ein Emulgatorsystem (B) mit einem HLB-Wert von 8 bis 18, das in einer Menge von mindestens 2 Gew.-%, bezogen auf das Gesamtgewicht der Zusammensetzung, enthalten ist,
- ein schaumbildendes System (C), das mindestens einen anionischen grenzflächenaktiven Stoff enthält, der unter den Alkylglycolcarbonsäuren und ihren Salzen der folgenden Formel (I) ausgewählt ist:
R₁-CHOH-CH₂-O-CH₂-COO⁻X⁺ (I),
worin R₁ eine gesättigte oder ungesättigte, geradkettige oder verzweigte Alkylgruppe mit 8 bis 30 Kohlenstoffatomen bedeutet und X Wasserstoff oder ein anorganisches oder organisches Kation ist,
- wobei das Gewichtsverhältnis Ölphase (A) / schaumbildendes System (C) im Bereich von 1,5 bis 12 liegt.

2. Zusammensetzung nach Anspruch 1, **dadurch gekennzeichnet, dass** sie durch die Technik Emulgieren unter Phaseninversion erhältlich ist.

3. Zusammensetzung nach Anspruch 1 oder 2, **dadurch gekennzeichnet, dass** die mittlere Tröpfchengröße der Ölphase D[4,3] im Bereich von 0,09 bis 4 µm liegt.

4. Zusammensetzung nach einem der vorhergehenden Ansprüche, **dadurch gekennzeichnet, dass** der anionische grenzflächenaktive Stoff eine Verbindung der Formel (I) ist, worin R₁ eine geradkettige oder verzweigte, gesättigte oder ungesättigte Alkylgruppe mit 8 bis 18 Kohlenstoffatomen bedeutet.

5. Zusammensetzung nach dem vorhergehenden Anspruch, **dadurch gekennzeichnet, dass** der anionische grenzflächenaktive Stoff, der unter den Alkylglycolcarbonsäuren und deren Salzen ausgewählt ist, das Natriumlaurylglycolcarboxylat oder seine Säureform ist.

6. Zusammensetzung nach einem der vorhergehenden Ansprüche, **dadurch gekennzeichnet, dass** das schaumbildende System ferner einen oder mehrere schaumbildende grenzflächenaktive Stoffe enthält, die unter den Alkylpolyglucosiden, Betainderivaten, Alkylphosphaten, Alkylethersulfaten und deren Gemischen ausgewählt sind.

7. Zusammensetzung nach einem der vorhergehenden Ansprüche, **dadurch gekennzeichnet, dass** der Mengenanteil des schaumbildenden Systems im Bereich von 0,5 bis 20 Gew.-%, bezogen auf das Gesamtgewicht der Zusammensetzung, liegt.

8. Zusammensetzung nach einem der vorhergehenden Ansprüche, **dadurch gekennzeichnet, dass** das Gewichtsverhältnis Ölphase (A) / schaumbildendes System (C) im Bereich von 4 bis 10 liegt.

9. Zusammensetzung nach einem der vorhergehenden Ansprüche, **dadurch gekennzeichnet, dass** der Emulgator unter den ethoxylierten Fettalkoholen, ethoxylierten Fettsäuren, partiellen Glyceriden von Fettsäuren, die ethoxyliert sind, Triglyceriden von Fettsäuren, die mehrfach mit Glycerin verethert sind, und ihren ethoxylierten Derivaten und deren Gemischen ausgewählt sind.

10. Zusammensetzung nach dem vorhergehenden Anspruch, **dadurch gekennzeichnet, dass** der Emulgator unter den Additionsprodukten von Ethylenoxid und Laurylalkohol; Additionsprodukten von Ethylenoxid und Behenylalkohol; Additionsprodukten von Ethylenoxid und Cetearylalkohol; Additionsprodukten von Ethylenoxid und Cetylalkohol; Additionsprodukten von Ethylenoxid und Stearylalkohol; Additionsprodukten von Ethylenoxid und Isostearylalkohol; Additionsprodukten von Ethylenoxid und Laurinsäure, Palmitinsäure, Stearinsäure oder Behensäure und deren Gemischen ausgewählt ist.

11. Zusammensetzung nach einem der vorhergehenden Ansprüche, **dadurch gekennzeichnet, dass** das Emulgatorsystem ferner einen oder mehrere Coemulatoren enthält, die unter den Fettalkoholen mit 8 bis 30 Kohlenstoffatomen; Fettsäuren mit 8 bis 30 Kohlenstoffatomen; Glycerinfettsäureestern; deren ethoxylierten Derivaten mit 2 bis 8 Ethylenoxidgruppen und ihren Gemischen ausgewählt sind.

12. Zusammensetzung nach einem der vorhergehenden Ansprüche, **dadurch gekennzeichnet, dass** der Mengenanteil des Emulgatorsystems im Bereich von 2 bis 20 Gew.-%, bezogen auf das Gesamtgewicht der Zusammensetzung, liegt.

13. Zusammensetzung nach einem der vorhergehenden Ansprüche, **dadurch gekennzeichnet, dass** das Verhältnis Emulgatorsystem (B) / Ölphase (A) im Bereich von 0,04 bis 0,2 liegt.

14. Zusammensetzung nach einem der vorhergehenden Ansprüche, **dadurch gekennzeichnet, dass** der Mengenanteil der Ölphase im Bereich von 30 bis 70 Gew.-%, bezogen auf das Gesamtgewicht der Zusammensetzung, liegt.

15. Zusammensetzung nach einem der vorhergehenden Ansprüche, **dadurch gekennzeichnet, dass** die Ölphase ein oder mehrere Öle enthält, die eine Molmasse von mindestens 360 g/mol aufweisen.

16. Zusammensetzung nach dem vorhergehenden Anspruch, **dadurch gekennzeichnet, dass** die Ölphase mindestens ein Öl enthält, das unter Vaselineöl, 2-Ethylhexylpalmitat, Cetyl-2-ethyl-hexanoat und deren Gemischen ausgewählt ist.

17. Zusammensetzung nach einem der vorhergehenden Ansprüche, **dadurch gekennzeichnet, dass** sie eine Wassermenge von höchsten 45 % des Gesamtgewichts der Zusammensetzung enthält.

18. Zusammensetzung nach einem der vorhergehenden Ansprüche, **dadurch gekennzeichnet, dass** es sich um ein Produkt zur Reinigung und/oder zum Abschminken der Haut, ein Produkt für die Dusche, ein Haarwaschmittel oder ein Haarpflegemittel, ein Produkt für die Rasur, eine Maske, die abgespült wird, ein exfoliatives Produkt handelt.

19. Kosmetische Verwendung einer kosmetischen Zusammensetzung nach einem der Ansprüche 1 bis 17 als Produkt für die Reinigung und/oder zum Schminken der Haut, als Produkt für die Dusche, als Haarwaschmittel, als Haarpflegeprodukt, als Produkt für die Rasur, als Maske, die abgespült wird, als exfoliatives Produkt.

20. Verfahren zur Reinigung einer Keratinsubstanz, **dadurch gekennzeichnet, dass** auf die Keratinsubstanz eine Zusammensetzung nach einem der Ansprüche 1 bis 17 aufgetragen und dann gespült wird.
